# EUROPEAN PATENT APPLICATION

(11) **EP 1 043 020 A1**
(43) Date of publication of application: **11.10.2000**
(21) Application number: 98961566.1
(22) Date of filing: 25.12.1998
(51) Int. Cl.: A61K 31/155, A61K 31/40, A61K 31/445, A61K 31/405, A61K 47/10, A61K 47/14, A61K 47/30

(54) **MEDICINAL COMPOSITION FOR PERCUTANEOUS ADMINISTRATION**

(30) Priority: 25.12.1997 JP 35715197
(71) Applicant: DAIICHI PHARMACEUTICAL CO., LTD., Chuo-ku, Tokyo 103-8234 (JP); Saitama Daiichi Pharmaceutical Co., Ltd., Saitama 344-0057 (JP)
(72) Inventor: IGARASHI, Kyoko, Saitama Daiichi Pharma. Co., Ltd., Kasukabe-shi, Saitama 344-0057 (JP); KAWAMURA, Naohisa, Saitama Daiichi Pharma. Co. Ltd, Kasukabe-shi, Saitama 344-0057 (JP)
(74) Representative: Wächtershäuser, Günter, Prof. Dr.
(86) International application number: JP9805919
(87) International publication number: WO9933458

(57) **Abstract**

A percutaneously absorbable medicinal composition comprising a percutaneous absorption enhancer and at least one member selected from the group consisting of aromatic amidine derivatives represented by formula (1): 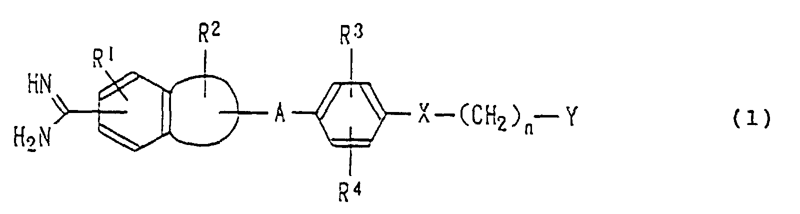 salts of the derivatives, solvates of the derivatives, and solvates of salts of the derivatives.

The present invention provides a composition which exhibits high percutaneous absorbability, which can maintain an effective blood level for prolonged periods of time, which provides antithrombotic and anticoagulant effects.

## Description

### Technical Field

The present invention relates to a percutaneously absorbable medicinal composition containing an aromatic amidine derivative which is useful as an anticoagulant or a prophylactic and therapeutic drug for thrombosis. Background Art

Japanese Patent Application Laid-Open (*kokai*) No. 5-208946 and International Patent Publication WO 96/16940 disclose that a compound having an aromatic amidine structure inhibits blood coagulation factor X and is useful as an anticoagulant or prophylactic and therapeutic drug for thrombosis.

Conventionally, drugs acting on the blood coagulation system are typically administered intravenously or perorally, and other types of administration have rarely been investigated.

In view of the foregoing, an object of the present invention is to provide a novel mode of administration of the aforementioned aromatic amidine derivatives.

### Disclosure of the Invention

The present inventors have conducted extensive studies on absorbability of the aforementioned aromatic amidine derivative through percutaneous administration. Quite surprisingly, the inventors have found that this compound is effectively absorbed from the skin and an effective blood level thereof can be maintained when the compound and a percutaneous absorption enhancer are used in combination. The present invention has been accomplished on the basis of this finding.

Accordingly, the present invention provides a medicinal composition for percutaneous administration comprising a percutaneous absorption enhancer and an aromatic amidine derivative represented by the following formula (1), a salt of the derivative, a solvate of the derivative, and a solvate of the salt of the derivative: 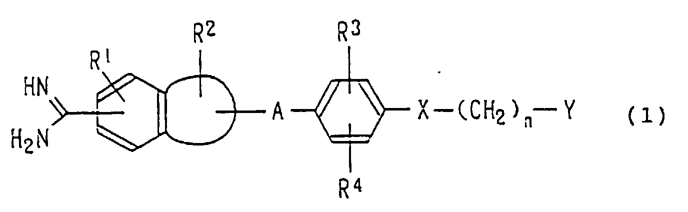 [wherein
R¹ represents a hydrogen atom or a lower alkoxyl group;
R² represents a hydrogen atom, a lower alkyl group, a lower alkoxyl group, a carboxyl group, an alkoxycarbonyl group, a carboxyalkyl group, or an alkoxycarbonylalkyl group;
R³ represents a hydrogen atom, a carboxyl group, an alkoxycarbonyl group, a carboxyalkyl group, an alkoxycarbonylalkyl group, a carboxyalkoxyl group or an alkoxycarbonylalkoxyl group;
R⁴ represents a hydrogen atom, a halogen atom, an amino group, a cyano group, a nitro group, a hydroxyl group, a lower alkyl group, or a lower alkoxyl group;
n represents an integer 0 to 4; and
A represents a C1-C4 alkylene group optionally substituted by one or two hydroxyalkyl groups, carboxyl groups, alkoxycarbonyl groups, carboxyalkyl groups, or alkoxycarbonylalkyl groups, or a group represented by the following formula; 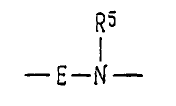 {wherein
E represents a lower alkylene group or a carbonyl group and R⁵ represents a hydrogen atom or a group represented by formula -D-W-R⁶ (wherein D is a group represented by 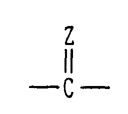 (wherein
Z is an oxygen atom or a sulfur atom), or a group represented by 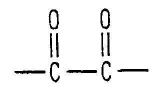 or a sulfonyl group;
W represents a single bond or a group represented by -NR⁷-(wherein R⁷ represents a hydrogen atom, a carbamoyl group, a lower alkoxycarbonyl group, a mono- or di-lower alkylaminocarbonyl group, a lower alkylsulfonyl group, a mono- or di-lower alkylaminothiocarbonyl group, a lower alkyl group which may have a substituent, or a lower alkanoyl group which may have a substituent);
R⁶ represents a hydroxyl group, a lower alkoxyl group, a lower alkyl group which may have a substituent, an aryl group which may have a substituent, or a heteroaryl group which may have a substituent)};
X represents a single bond, an oxygen atom, a sulfur atom, or a carbonyl group;
Y represents a saturated or unsaturated 5- or 6-membered heterocyclic or cyclic hydrocarbon group which may have a substituent, an amino group which may have a substituent, or an aminoalkyl group which may have a substituent; and the group represented by 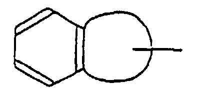 represents a group selected from among indolyl, benzofuranyl, benzothienyl, benzimidazolyl, benzoxazolyl, benzothiazolyl, naphthyl, tetrahydronaphthyl, or indanyl].

### Best Modes for Carrying Out the Invention

As described above, the aromatic amidine derivatives (1), salts of the derivatives, solvates of the derivatives, and solvates of the salts of the derivatives which are used in the composition of the present invention are known to inhibit blood coagulation factor X and thus are useful as anticoagulants or prophylactic and therapeutic agents for thrombosis (Japanese Patent Application Laid-Open (*kokai*) No. 5-208946 and International Patent Publication WO 96/16940).

In the above-described formula (1), examples of the lower alkyl group include C1-C6 linear, branched, and cyclic alkyl groups. Specific examples thereof include a methyl group, an ethyl group, a propyl group, an isopropyl group, a butyl group, a secondary butyl group, a tertiary butyl group, a pentyl group, a hexyl group, a cyclopropyl group, a cyclobutyl group, a cyclopentyl group, and a cyclohexyl group.

The lower alkyl group may have a substituent, and examples of the substituent include a halogen atom, a carboxyl group, a carbamoyl group, an amino group, a cyano group, a nitro group, a lower alkanoyl group, a lower alkoxyl group, a lower alkoxycarbonyl group, a mono- or di- lower alkylamino group, an aryl group, an aralkyloxy group, an aryloxy group, a mercapto group, a lower alkylthio group, a lower alkylthiocarbonyl group, a hydroxyl group, a carbamoyl group, and a mono- or di- lower alkylaminocarbonyl group.

Examples of the lower alkoxyl groups include a C1-C6 alkoxyl group, and specific examples include a methoxyl group, an ethoxyl group, a propoxyl group, an isopropoxyl group, a butoxyl group, a secondary butoxyl group, and a tertiary butoxyl group.

Examples of the alkoxycarbonyl groups include a methoxycarbonyl group, an ethoxycarbonyl group, a propoxycarbonyl group, and a butoxycarbonyl group.

Examples of the carboxyalkyl groups include a carboxymethyl group, a carboxyethyl group, and a carboxypropyl group.

Examples of the alkoxycarbonylalkyl groups include a methoxycarbonylmethyl group, an ethoxycarbonylmethyl group, a proxycarbonylmethyl group, a methoxycarbonylethyl group, ethoxycarbonylethyl group, a methoxycarbonylpropyl group, and an ethoxycarbonylpropyl group.

Examples of the carboxyalkoxyl groups include a carboxymethoxyl group, a carboxyethoxyl group, and a carboxypropoxyl group. Examples of the alkoxycarbonylalkoxyl group include a methoxycarbonylmethoxyl group, an ethoxycarbonylmethoxyl group, a propoxycarbonylmethoxyl group, a methoxycarbonylethoxyl group, and an ethoxycarbonylethoxyl group.

Examples of the hydroxyalkyl groups include a hydroxymethyl group, a hydroxyethyl group, a hydroxypropyl group, and a hydroxybutyl group. Examples of the C1-C4 alkylene groups include a methylene group, an ethylene group, a trimethylene group, and a tetramethylene group.

Examples of the mono- or di- lower alkylaminocarbonyl groups include mono-lower alkylaminocarbonyl groups such as a methylaminocarbonyl group, an ethylaminocarbonyl group, a propylaminocarbonyl group, an isopropylaminocarbonyl group, a butylaminocarbonyl group, an isobutylaminocarbonyl group, a pentylaminocarbonyl group, an isopentylaminocarbonyl group, a hexylaminocarbonyl group, and an isohexylaminocarbonyl group. Examples of the di-alkylaminocarbonyl groups include symmetric dialkylaminocarbonyl group having two lower alkyl groups as substituents such as a dimethylaminocarbonyl group, a diethylaminocarbonyl group, a dipropylaminocarbonyl group, a diisopropylaminocarbonyl group, a dibutylaminocarbonyl group, and a dipentylaminocarbonyl group; and asymmetric dialkylaminocarbonyl groups having two different lower alkyl groups as substituent such as an ethylmethylaminocarbonyl group, a methylpropylaminocarbonyl group, an ethylpropylaminocarbonyl group, a butylmethylaminocarbonyl group, a butylethylaminocarbonyl group, and a butylpropylaminocarbonyl group.

Examples of the lower alkylsulfonyl groups include a methylsulfonyl group, an ethylsulfonyl group, a propylsulfonyl group, an isopropylsulfonyl group, a butylsulfonyl group, an isobutylsulfonyl group, a pentylsulfonyl group, an isopentylsulfonyl group, a hexylsulfonyl group, and an isohexylsulfonyl group.

With regard to the mono- or di-lower alkylaminothiocarbonyl groups, examples of the monoloweralkylaminothiocarbonyl groups include a methylaminothiocarbonyl group, an ethylaminothiocarbonyl group, a propylaminothiocarbonyl group, an isopropylaminothiocarbonyl group, a butylaminothiocarbonyl group, an isobutylaminothiocarbonyl group, a pentylaminothiocarbonyl group, an isopentylaminothiocarbonyl group, a hexylaminothiocarbonyl group, or an isohexylaminothiocarbonyl group. Examples of the dialkylaminothiocarbonyl group include symmetric dialkylaminothiocarbonyl group di-substituted by lower alkyl groups such as a dimethylaminothiocarbonyl group, a diethylaminothiocarbonyl group, a dipropylaminothiocarbonyl group, a diisopropylaminothiocarbonyl group, a dibutylaminothiocarbonyl group, or a dipentylaminothiocarbonyl group and asymmetric dialkylaminotiocarbonyl groups di-substituted by different lower alkyl groups such as an ethylmethylaminothiocarbonyl group, a methylpropylaminothiocarbonyl group, an ethylpropylaminothiocarbonyl group, a butylmethylaminothiocarbonyl group, a butylethylaminothiocarbonyl group, or a butylpropylaminothiocarbonyl group.

Examples of the lower alkanoyl groups include a formyl group, an acetyl group, a propionyl group, a butyryl group, an isobutyryl group, a valeryl group, an isovaleryl group, a pivaloyl group, and a hexanoyl group. Of these, an acetyl group, a propionyl group and a butyryl group are preferred, with an acetyl group and a propionyl group being more preferred. The lower alkanoyl group may have a substituent.

Examples of groups which may serve as a substituent for the alkanoyl group include a halogen atom, a carboxyl group, a carbamoyl group, an amino group, a cyano group, a nitro group, a lower alkanoyl group, a lower alkoxyl group, a lower alkoxycarbonyl group, a mono- or di- lower alkylamino group, an aryl group, an aralkyloxy group, an aryloxy group, a mercapto group, a lower alkylthio group, a lower alkylthiocarbonyl group, a hydroxyl group, a carbamoyl group, or a mono- or di- lower alkylaminocarbonyl group.

Examples of the aryl groups include a phenyl group, a naphthyl group, a biphenyl group, and an anthryl group. The aryl group may have a substituent.

Examples of the heteroaryl groups include a furyl group, a thienyl group, a pyrolyl group, an imidazolyl group, a pyrazolyl group, an isothiazolyl group, an isoxazolyl group, a pyridyl group, a pyrimidinyl group, a quinolyl group, an isoquinolyl group, a quinazolinyl group, a quinolidinyl group, a quinoxalinyl group, a cinnolinyl group, a benzimidazolyl group, an imidazopyridyl group, a benzofuranyl group, a naphthylidinyl group, a 1,2-benzoisoxazolyl group, a benzoxazolyl group, a benzothiazolyl group, an oxazolopyridyl group, an isothiazolopyridyl group, and a benzothienyl group. Of these, a furyl group, a thienyl group, a pyrolyl group, an imidazolyl group, and a pyridyl group are preferred. The aryl group may have a substituent.

Examples of groups which may serve as a substituent of these aryl or heteroaryl groups include a halogen atom, a carboxyl group, an amino group, a cyano group, a nitro group, a hydroxyl group, a lower alkoxyl group, a lower alkoxycarbonyl group, a mono- or di-lower alkylamino group, a lower alkanoyl group, and a lower alkyl group optionally having a substituent.

Preferably, the saturated or unsaturated 5- or 6-membered heterocyclic group is a heterocyclic group having 1 or 2 nitrogen or oxygen atoms. Specific examples of the heterocycles include pyrrolidine, piperidine, imidazoline, piperazine, tetrahydrofuran, hexahydropyrimidine, pyrrole, imidazole, pyrazine, pyrrolidinone, piperidinone, and morpholine. Of these, pyrrolidine and piperidine are particularly preferred. Examples of the saturated or unsaturated cyclic hydrocarbon groups include a cyclopentyl group and a cyclohexyl group. Examples of the aminoalkyl groups include an aminomethyl group, an aminoethyl group, and an aminopropyl group.

The heterocyclic groups and cyclic hydrocarbon groups may have a substituent. Examples of the groups which may serve as a substituent of the heterocyclic groups and cyclic hydrocarbon groups include a lower alkyl group, a lower alkanoyl group, a carbamoyl group, a monoalkylcarbamoyl group, a dialkylcarbamoyl group, a formimidoyl group, an alkanoimidoyl group, a benzimidoyl group, a carboxyl group, an alkoxycarbonyl group, a carboxyalkyl group, an alkylcarbonylalkyl group, an aminoalkyl group, an alkanoylamino group, an alkanoylaminoalkyl group, an imino group, and an alkoxycarbonylimino group.

Examples of groups which may serve as a substituent of the amino group and aminoalkyl group include a lower alkyl group, a pyrrolidinyl group, a pyrazyl group, a carbamoyl group, a monoalkylcarbamoyl group, a dialkylcarbamoyl group, a lower alkanoyl group, a formimidoyl group, an alkanoimidoyl group, a benzimidoyl group, and an alkoxycarbonyl group.

The above-described groups such as an alkyl group, an alkoxyl group, and an alkanoyl group and moieties in the substituents such as an alkyl moiety, an alkoxyl moiety, or an alkanoyl moiety preferably have 1 to 6 carbon atoms.

Particularly preferable examples of the group represented by the following formula: 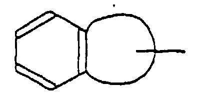 include a group selected from among benzofuranyl, benzimidazolyl, indolyl, benzothienyl, benzothiazolyl, naphthyl, and tetrahydronaphthyl.

The aromatic amidine derivatives represented by formula (1) according to the present invention, salts of the derivatives, solvates of the derivatives, and solvates of the salts of the derivatives may have an asymmetric carbon atom. In this case, optical isomers, stereoisomers, and mixtures thereof attributed to the asymmetric carbon atom are all within the scope of the present invention.

In the present invention, among the above-described aromatic amidine derivatives (1), salts of the derivatives, solvates of the derivatives, and solvates of the salts of the derivatives, the following compounds are particularly preferred:
2-[4-[((3S)-1-acetimidoyl-3-pyrrolidinyl)oxy]phenyl]-3-(7-amidino-2-naphthyl)propionic acid,
(+)-2-[4-[((3S)-1-acetimidoyl-3-pyrrolidinyl)oxy]phenyl]-3-(7-amidino-2-naphthyl)propionic acid,
(2S)-2-[4-[((3S)-1-acetimidoyl-3-pyrrolidinyl)oxy]phenyl]-3-(7-amidino-2-naphthyl)propionic acid,
(2R)-2-[4-[((3R)-1-acetimidoyl-3-pyrrolidinyl)oxy]phenyl]-3-(7-amidino-2-naphthyl)propionic acid,
2-[4-[(1-acetimidoyl-4-piperidyl)oxy]phenyl]-3-(7-amidino-2-naphthyl)propionic acid,
(+)-2-[4-[(1-acetimidoyl-4-piperidyl)oxy]phenyl]-3-(7-amidino-2-naphthyl)propionic acid,
2-[4-[(1-acetimidoyl-4-piperidyl)oxy]phenyl]-3-(5-amidinobenzo[b]thien-2-yl)propionic acid,
2-[4-[((2S)-1-acetimidoyl-2-pyrrolidinyl)methoxy]phenyl]-3-(5-amidinobenzo[b]thien-2-yl)propionic acid,
(+)-2-[4-[((2S)-1-acetimidoyl-2-pyrrolidinyl)methoxy]phenyl]-3-(5-amidinobenzo[b]thien-2-yl)propionic acid,
3-[4-[((3S)-1-acetimidoyl-3-pyrrolidinyl)oxy]phenyl]-4-(5-amidinobenzo[b]thien-2-yl)butyric acid,
2-[4-[((3S)-1-acetimidoyl-3-pyrrolidinyl)oxy]phenyl]-3-(6-amidino-1-ethyl-2-indolyl)propionic acid,
2-[4-[((3R)-1-acetimidoyl-3-pyrrolidinyl)oxy]phenyl]-3-(6-amidino-1-ethyl-2-indolyl)propionic acid.
2-[4-[(1-acetimidoyl-4-piperidyl)oxy]phenyl]-3-(6-amidino-1-ethyl-2-indolyl)propionic acid,
N-[4-[(1-acetimidoyl-4-piperidyl)oxy]phenyl]-N-[(7-amidino-2-naphthyl)methyl]-N'-methylsulfamide,
ethyl N-[N-4-[(1-acetimidoyl-4-piperidyl)oxy]phenyl]-N-[(7-amidino-2-naphthyl)methyl]sulfamoyl]carbamate,
4-[N-4-[(1-acetimidoyl-4-piperidyl)oxy]phenyl]-N-[(7-amidino-2-naphthyl)methyl]sulfamoyl]benzoic acid,
N-[4-[(1-acetimidoyl-4-piperidyl)oxy]phenyl]-N-[(7-amidino-2-naphthyl)methyl]sulfamoylacetic acid,
ethyl N-[N-[4-[(1-acetimidoyl-4-piperidyl)oxy]phenyl]-N-[(7-amidino-2-naphthyl)methyl]sulfamoyl]glycinate,
N-[N-4-[(1-acetimidoyl-4-piperidyl)oxy]phenyl]-N-[(7-amidino-2-naphthyl)methyl]sulfamoyl]-N-ethoxycarbonylglycine, and
N-[N-4-[(1-acetimidoyl-4-piperidyl)oxy]phenyl]-N-[(7-amidino-2-naphthyl)methyl]sulfamoyl]glycine.

Particularly preferred ones are:
(2S)-2-[4-[((3S)-1-acetimidoyl-3-pyrrolidinyl)oxy]phenyl]-3-(7-amidino-2-naphthyl)propionic acid,
(+)-2-[4-[(1-acetimidoyl-4-piperidyl)oxy]phenyl]-3-(7-amidino-2-naphthyl)propionic acid,
(+)-2-[4-[((2S)-1-acetimidoyl-2-pyrrolidinyl)methoxy]phenyl]-3-(5-amidinobenzo[b]thien-2-yl)propionic acid,
ethyl N-[N-[4-[(1-acetimidoyl-4-piperidyl)oxy]phenyl]-N-[(7-amidino-2-naphthyl)methyl]sulfamoyl]glycinate,
N-[N-4-[(1-acetimidoyl-4-piperidyl)oxy]phenyl]-N-[(7-amidino-2-naphthyl)methyl]sulfamoyl]glycine, and
N-[4-[(1-acetimidoyl-4-piperidyl)oxy]phenyl]-N-[(7-amidino-2-naphthyl)methyl]sulfamoylacetic acid.

Furthermore, the following compounds are also preferred:
(2S)-2-[4-[((3S)-1-acetimidoyl-3-pyrrolidinyl)oxy]phenyl]-3-(7-amidino-2-naphthyl)propionic acid hydrochloride pentahydrate, (+)-2-[4-[(1-acetimidoyl-4-piperidyl)oxy]phenyl]-3-(7-amidino-2-naphthyl)propionic acid dihydrochloride,
(+)-2-[4-[((2S)-1-acetimidoyl-2-pyrrolidinyl)methoxy]phenyl]-3-(5-amidinobenzo[b]thien-2-yl)propionic acid dihydrochloride,
ethyl N-[N-[4-[(1-acetimidoyl-4-piperidyl)oxy]phenyl]-N-[(7-amidino-2-naphthyl)methyl]sulfamoyl]glycinate dihydrochloride,
N-[N-4-[(1-acetimidoyl-4-piperidyl)oxy]phenyl]-N-[(7-amidino-2-naphthyl)methyl]sulfamoyl]glycine dihydrochloride, and
N-[4-[(1-acetimidoyl-4-piperidyl)oxy]phenyl]-N-[(7-amidino-2-naphthyl)methyl]sulfamoylacetic acid dihydrochloride.

Examples of substances which enhance percutaneous absorption of the aforementioned amidine derivatives (1), salts of the derivatives, solvates of the derivatives, or solvates of the salts of the derivatives (i.e., examples of percutaneous absorption enhancers) include one or more species selected from among alcohols, polyhydric alcohols, higher alkanes, higher fatty acids, higher fatty acid esters, terpenes, alkyl sulfate esters, alkylamine oxides, carboxybetaines, polyoxyalkylene alkyl ethers, sulfoxides, and amides. Of these, there may be preferably used one or more species, singly or in combination, selected from among alcohols, polyhydric alcohols, higher fatty acids, higher fatty acid esters, terpenes, alkyl sulfate esters, alkylamine oxides, carboxybetaines, polyoxyalkylene alkyl ethers, and amides.

Among the alcohols serving as the aforementioned percutaneous absorption enhancers, C2-C18 saturated aliphatic alcohols and C7-C14 arylalkanols are preferred. Specific examples include C2-C4 alcohols such as ethanol and isopropanol; C7-C12 aryl alcohols such as benzyl alcohol; 1-octanol; 1-nonanol; 1-decanol; 1-dodecanol; and stearyl alcohol.

Examples of polyhydric alcohols include C2-C4 alkylene glycols, glycerin, polyethylene glycol, polyglycerin, and sorbitan. Specific examples include ethylene glycol, propylene glycol, butylene glycol, glycerin, polyethylene glycol, and sorbitan.

Among higher alkanes, C6-C12 alkanes are preferred. Examples include n-heptane, n-nonane, and n-dodecane.

Among higher fatty acids, C6-C18 saturated or unsaturated fatty acids are preferred, with C6-C16 saturated or unsaturated fatty acids being more preferred. Examples of higher fatty acids include caproic acid, caprylic acid, capric acid, lauric acid, myristic acid, and oleic acid.

Among higher fatty acid esters, C6-C24 saturated or unsaturated fatty acid esters of monohydric or polyhydric alcohol are preferred, with C6-C24 fatty acid esters of polyhydric alcohol being preferred, C6-C16 fatty acid esters of polyhydric alcohol being more preferred. Examples of polyhydric alcohols include ethylene glycol, polyethylene glycol, propylene glycol, polypropylene glycol, glycerin, polyglycerin, and sorbitan. Examples of fatty acids which form esters with polyhydric alcohol include caproic acid, caprylic acid, capric acid, lauric acid, and myristic acid.

Among these polyhydric alcohol fatty acid esters, polyhydric alcohol monofatty acid esteres are preferred, with polyhydric alcohol mono C6-C16 fatty acid being more preferred, glycerin monofatty acid esters being further preferred, glycerin mono C6-C16 fatty acid esters being particularly preferred. Specific examples of glycerin monofatty acid esters include glycerin monocaproate, glycerin monocaprylate, and glycerin monocaprate, with glycerin monocaprylate being particularly preferred.

Examples of terpenes include monoterpenes and sesquiterpenes. Specific examples include cineole, 1-menthol, menthone, d-limonene, and nerolidol.

Examples of preferred alkyl sulfate esters include C6-C24 alkyl sulfate ester salts such as decyl sulfate salts, lauryl sulfate salts, and tetradecyl sulfate salts. Of these, lauryl sulfate alkali metal salts are more preferred, with sodium lauryl sulfate being particularly preferred.

Among alkylamine oxides, alkyldimethylamine oxides are preferred, with C6-C24 alkyldimethylamine oxides being more preferred. Specific examples include decyldimethylamine oxide, lauryldimethylamine oxide, and tetradecyldimethylamine oxide.

Among carboxybetaines, alkyldimethylaminoacetic acids are preferred, with C6-C24 alkyldimethylaminoacetic acids being more preferred. Specific examples include decyldimethylaminoacetic acid, dodecyldimethylaminoacetic acid, and tetradecyldimethylaminoacetic acid.

Examples of polyoxyalkylene alkyl ethers include polyoxyethylene alkyl ethers and polyoxypropylene alkyl ethers. Of these, polyoxyethylene alkyl ethers are preferred, with polyoxyethylene C6-C24 alkyl ether being particularly preferred. Examples of sulfoxides include dialkyl sulfoxides, e.g., dimethyl sulfoxide. Examples of amides include N,N-dialkylformamide, N,N-dialkylacetamide, N,N-dialkyltoluamide, N-hydroxyalkylactamide, pyrrolidone, and N-alkylpyrrolidone. Specific examples include N,N-dimethylformamide, N,N-dimethylacetamide, N,N-diethyltoluamide, N-hydroxyethylactamide, 2-pyrrolidone, and N-octyl-2-pyrrolidone.

Among the aforementioned percutaneous enhancers, there may be preferably used one or more species, singly or in combination, selected from among C2-C4 alcohols, C7-C12 arylalkanols, C6-C18 saturated aliphatic alcohols, polyhydric alcohols, C6-C16 fatty acids, terpenes, polyhydric alcohol mono C6-C16 fatty acid esters, C6-C24 alkyl sulfate salts, C6-C24 alkyldimethylamine oxides, C6-C24 alkyldimethylaminoacetic acids, polyethylene glycol C6-C24 alkyl ethers, pyrrolidone, N-alkylpyrrolidones, N,N-dialkylformamide, and N,N-dialkyltoluamides.

More preferably, there may be used one or more species, singly or in combination, selected from among ethanol, isopropanol, benzyl alcohol, ethylene glycol, propylene glycol, glycerin, 1-nonanol, 1-decanol, stearyl alcohol, caproic acid, caprylic acid, capric acid, d-limonene, cineole, propylene glycol monocaprylate, glycerin monocaprylate, glycerin monocaprate, lauryldimethylamine oxide, dodecyl-N,N-dimethylaminoacetic acid, sodium lauryl sulfate, polyoxyethylene lauryl ether, N,N-diethyltoluamide, 2-pyrrolidone, and N-octyl-2-pyrrolidone.

The following are also preferred: one or more species selected from among C2-C4 alcohols, C7-C12 arylalkanols, C6-C18 saturated aliphatic alcohols, C6-C16 fatty acids, terpenes, pyrrolidone, and N-alkylpyrrolidone.

More specifically, mention may be given of a single species or a combination of two to four species selected from among ethanol, capric acid, benzyl alcohol, 1-nonanol, N-octyl-2-pyrroridone, and d-limonene. Examples of combinations of these species include ethanol and capric acid; benzyl alcohol and capric acid; benzyl alcohol, and d-limonene; ethanol, capric acid, and 1-nonanol; ethanol, capric acid, and d-limonene; ethanol, benzyl alcohol, and 1-nonanol; ethanol, benzyl alcohol, and d-limonene; ethanol, N-octyl-2-pyrrolidone, and capric acid; ethanol, benzyl alcohol, 1-nonanol, and capric acid; capric acid, benzyl alcohol, d-limonene, and ethanol; and capric acid, benzyl alcohol, d-limonene, 1-nonanol, and ethanol.

In the medicinal composition for percutaneous administration of the present invention, no particular limitation is imposed on the compositional ratio of amidine derivatives (1), salts of the derivatives, solvates of the derivatives, or solvates of the salts of the derivatives to a percutaneous absorption enhancer. The ratio based on weight is preferably 0.01-100, more preferably 0.1-10, particularly preferably 0.2-5.

The amounts of amidine derivatives (1), salts of the derivatives, solvates of the derivatives, or solvates of the salts of the derivatives which are incorporated into the composition of the present invention depends on the dosage form. The amounts of incorporation are preferably 0.0001-60 wt.%, more preferably 0.001-50 wt.%, particularly preferably 0.1-30 wt.%. The total amount of percutaneous absorption enhancers is preferably 0.0001-60 wt.%, more preferably 0.001-50 wt.%, particularly preferably 0.1-50 wt.%. When two kinds of percutaneous absorption enhancers are used in combination, the ratio (on a weight basis) between two species is not particularly limited, and it is preferably 1/50 - 50/1.

The dosage form of the composition of the present invention is not particularly limited so long as it permits percutaneous absorption. For example, ointments, creams, gels, dermatological pastes, poultice, cataplasmas, plasters, patches, reservoir-type patches, lotions, or liniments may be employed. Examples of plasters include a matrix-type plaster comprising a tackifier such as acrylic resin, ethylene-vinyl acetate copolymer resin, or styrene-isobutylene-styrene block copolymer resin in which a drug and a percutaneous absorption enhancer are dissolved or dispersed. Examples of reservoir-type patches include one holding a liquid or semi-solid drug between a drug-releasing layer and a backing material layer which does not permit permeation of the drug, with a pressure-sensitive adhesive being provided at, for example, a peripheral portion. When these drugs are prepared, a variety of conventionally known pharmaceutically acceptable carriers (additives) may optionally be incorporated.

Examples of these additives include base materials for external use, emulsifiers, suspending agents, preservatives, stabilizers, and water.

The composition of the present invention may be formed into a variety of the aforementioned dosage forms through methods provided therefor, and in use, may be applied to the skin with optional massage or in the form of patch.

### Examples

The present invention will next be described in more detail by way of examples, which should not be construed as limiting the invention thereto.

### Test Example 1 (in vitro skin permeability test in rat)

Tests were performed under the following conditions, and there was calculated a cumulative amount of (2S)-2-[4-[((3S)-1-acetimidoyl-3-pyrrolidinyl)oxy]phenyl]-3-(7-amidino-2-naphthyl)propionic acid hydrochloride pentahydrate (hereinafter the compound will be referred to as "compound A") which permeates through the skin from each donor solution or suspension per unit area for 24 hours (hereinafter the amount will be referred to as "skin permeation amount") (Q: µg/cm²), for comparison of skin permeability (n=3).

### 〈Test conditions〉

Skin: a piece of the skin excised from the abdomen of Wistar male rat (eight-week age)
Apparatus: side by side (2-chamber) cell (volume: 0.8 mL, effective area: 0.2827 cm²) (37°C)
Donor side; a solution containing compound A (50 mg/mL) or a suspension (saline) and a different enhancer
Receiver side; a phosphate buffer (0.05 mol/L) containing a solution of benzalkonium chloride (0.01%) (pH: 6.8)
Preparation of sample solution: A receiver solution (0.3 mL) is sampled at predetermined points in time. To the thus-sampled solution (precise volume: 0.15 mL), a methanol solution (0.15 mL) of methyl p-hydroxybezoate which had been precisely measured was added, to thereby prepare a sample solution. The skin permeation amount of compound A is measured by means of HPLC.

The results are shown in Table 1. In Table 1, the ratio of the cumulative skin permeation amount of compound A in the case of incorporation of a percutaneous absorption enhancer to that in the case without the enhancer is shown as "percutaneous absorption enhancing effect."

**Table 1**

| Percutaneous absorption enhancers | Q₂₄(µg/cm² ± S.E.) | Percutaneous absorption enhancing effect |
|---|---|---|
| None | 709.05 ± 76.18 | 1.0 |
| 1-Nonanol | 15282.69 ± 2433.50 | 21.6 |
| 1-Decanol | 10200.25 ± 1049.70 | 14.4 |
| 1-Dodecanol | 2796.50 ± 716.82 | 3.9 |
| n-Heptane | 2750.84 ± 389.54 | 3.9 |
| n-Nonane | 2545.30 ± 712.63 | 3.6 |
| Caproic acid | 17697.37 ± 2094.15 | 25.0 |
| Caprylic acid | 10566.09 ± 2209.14 | 14.9 |
| Capric acid | 18796.65 ± 625.34 | 26.5 |
| Lauric acid | 17203.18 ± 2588.98 | 24.3 |
| Propylene glycol monocaprylate | 12093.92 ± 2946.30 | 17.1 |
| Glycerin monocaprylate | 18439.60 ± 419.73 | 26.0 |
| Cineole | 5411.22 ± 187.55 | 7.6 |
| 1-Menthol | 4339.38 ± 886.18 | 6.1 |
| Menthone | 3510.57 ± 739.55 | 5.0 |
| d-Limonene | 5121.85 ± 687.87 | 7.2 |
| Nerolidol | 4042.17 ± 740.63 | 5.7 |
| Sodium lauryl sulfate (10 mmol/L) | 1540.48 ± 591.22 | 2.2 |
| Lauryldimethylamine oxide (20 mmol/L) | 28070.51 ± 4963.79 | 39.6 |
| Dodecyl-N,N-dimethylaminoacetic acid | 21958.07 ± 776.70 | 31.0 |
| Polyoxyethylene lauryl ether | 8687.2 ± 4652.2 | 10.5 |
| Lauryldimethylamine oxide (20 mmol/L) + glycerin monocaprylate (1%) | 6979.22 ± 395.00 | 9.4 |
| Lauryldimethylamine oxide (20 mmol/L) + glycerin monocaprylate (5%) | 17918.72 ± 2598.04 | 24.1 |
| Lauryldimethylamine oxide (20 mmol/L) + limonene (10 mmol/L) | 15422.01 ± 687.43 | 20.8 |
| Lauryldimethylamine oxide (20 mmol/L) + limonene (20 mmol/L) | 10724.49 ± 1253.28 | 14.4 |
| Lauryldimethylamine oxide (20 mmol/L) + polyoxyethylene lauryl ether (5%) | 4527.66 ± 547.57 | 6.1 |
| Sodium lauryl sulfate (10 mmol/L) + glycerin monocaprylate (5%) | 36459.58 ± 8771.09 | 44.2 |

### Test Example 2 (in vitro skin permeability test in pig)

The procedure of Test Example 1 was repeated, except that a piece of the skin excised from the back of a Yucatan miniature pig (five-month age) was employed, to thereby calculate the cumulative skin permeation amount of compound A per unit area for 48 hours (µg/cm²), for comparison of skin permeability (n=3).

The results are shown in Tables 2 to 4. In Tables 2 to 4, the ratio of the cumulative skin permeation amount of compound A in the case of incorporation of a percutaneous absorption enhancer to that in the case without the enhancer is shown as "percutaneous absorption enhancing effect."

**Table 2**

| Percutaneous absorption enhancers | Q₄₈(µg/cm² ± S.E.) | Percutaneous absorption enhancing effect |
|---|---|---|
| None | 61 ± 32 | 1.0 |
| Capric acid (0.5%) + ethylene glycol (5%) | 946 ± 60 | 15.5 |
| Capric acid (0.5%) + benzyl alcohol (5%) | 1244 ± 222 | 20.4 |
| Capric acid (0.5%) + ethanol (5%) | 984 ± 353 | 16.1 |
| Capric acid (0.5%) + 1-nonanol (5%) | 1450 ± 323 | 23.8 |
| Capric acid (5%) + 1-nonanol (0.5%) | 1442 ± 448 | 23.6 |
| Capric acid (5%) + d-limonene (5%) | 2328 ± 357 | 38.2 |
| Capric acid (5%) + polyoxyethylene lauryl ether (5%) | 1579 ± 651 | 25.9 |
| Capric acid (5%) + ethanol (5%) | 3637 ± 1286 | 59.6 |
| Capric acid (5%) + d-limonene (5%) + polyoxyethylene lauryl ether (5%) | 939 ± 278 | 15.4 |
| Capric acid (5%) + lauryldimethylamine oxide (0.5%) + 1-nonanol (0.5%) | 1812 ± 264 | 29.7 |
| Capric acid (5%) + N,N-dimethylformamide (5%) | 1458 ± 496 | 23.9 |
| Capric acid (5%) + 2-pyrrolidone (5%) | 1416 ± 425 | 23.2 |
| Capric acid (5%) + N-octyl-2-pyrrolidone (5%) Capric acid (5%) + 1-nonanol (0.5%) + | 1586 ± 599 | 26.0 |
| lauryldimethylamine oxide (0.5%) + benzyl alcohol (5%) | 1486 ± 593 | 24.4 |
| 1-Nonanol (0.5%) | 1497 ± 225 | 24.5 |
| 1-Nonanol (0.5%) + benzyl alcohol (5%) | 5233 ± 1955 | 85.8 |
| 1-Nonanol (0.5%) + ethanol (5%) | 1764 ± 292 | 28.9 |
| Benzyl alcohol (5%) + d-limonene (5%) | 4662 ± 7 | 76.4 |
| Lauryldimethylamine oxide (0.5%) | 1098 ± 263 | 18.0 |
| Lauryldimethylamine oxide (5%) | 1168 ± 410 | 19.1 |
| Lauryldimethylamine oxide (0.5%) + benzyl alcohol (5%) | 3360 ± 512 | 55.1 |
| Ethanol (20%) + d-limonene (5%) + capric acid (0.5%) | 3171 ± 905 | 52.0 |
| Ethanol (20%) + capric acid (0.5%) | 1263 ± 149 | 20.7 |

**Table 3**

| Percutaneous absorption enhancers | Q₄₈(µg/cm² ± S.E.) | Percutaneous absorption enhancing effect |
|---|---|---|
| None | 61 ± 32 | 1.0 |
| Ethanol (20%) + 1-nonanol (0.5%) + benzyl alcohol (0.5%) | 2251 ± 399 | 36.9 |
| Ethanol (20%) + capric acid (0.5%) + benzyl alcohol (0.5%) + d-limonene (5%) | 5251 ± 1111 | 86.1 |
| Ethanol (20%) + capric acid (0.5%) + benzyl alcohol (0.5%) + 1-nonanol (0.5%) | 5843 ± 517 | 95.8 |
| Ethanol (20%) + capric acid (0.5%) + benzyl alcohol (0.5%) + d-limonene (5%) + 1-nonanol (0.5%) | 6239 ± 1355 | 102.3 |
| Ethanol (20%) + capric acid (0.5%) + d-limonene (5%) + 1-nonanol (0.5%) | 4457 ± 710 | 73.1 |
| Ethanol (20%) + capric acid (0.5%) + 1-nonanol (0.5%) | 10060 ± 1600 | 164.9 |
| Ethanol (20%) + d-limonene (5%)+ 1-nonanol (0.5%) | 1985 ± 166 | 32.5 |
| Ethanol (20%) + 1-nonanol (0.5%) | 3052 ± 583 | 50.0 |

**Table 4**

| Percutaneous absorption enhancers | Q₄₈(µg/cm² ± S.E.) | Percutaneous absorption enhancing effect |
|---|---|---|
| None | 22 ± 10 | 1.0 |
| Capric acid (0.5%) + benzyl alcohol (0.5%) + d-limonene (5%) | 650 ± 165 | 29.5 |
| Capric acid (0.5%) + benzyl alcohol (0.5%) + 1-nonanol (0.5%) | 1268 ± 793 | 57.7 |
| Capric acid (0.5%) + benzyl alcohol (0.5%) + 1-nonanol (0.5%) + d-limonene (5%) | 698 ± 195 | 31.8 |
| Ethanol (20%) + capric acid (5%) | 1986 ± 339 | 90.3 |
| Ethanol (20%) + capric acid (5%) + polyoxyethylene lauryl ether (9E.O.)(5%) | 1812 ± 359 | 82.4 |
| Ethanol (20%) + capric acid (5%) + dimethylformamide (5%) | 2343 ± 440 | 106.5 |
| Ethanol (20%) + capric acid (5%) + 2-pyrrolidone (5%) | 3128 ± 833 | 142.2 |
| Ethanol (20%) + capric acid (5%) + N-octyl-2-pyrrolidone (5%) | 4116 ± 466 | 187.1 |
| Ethanol (20%) + capric acid (5%) + d-limonene (5%) | 2746 ± 704 | 124.8 |
| Ethanol (20%) + benzyl alcohol (5%) + d-limonene (5%) | 3513± 1517 | 159.7 |
| Ethanol (20%) + capric acid (0.5%) + benzyl alcohol (0.5%) + d-limonene (5%) | 1798 ± 551 | 81.7 |
| Ethanol (20%) + capric acid (0.5%) + benzyl alcohol (0.5%) + 1-nonanol (0.5%) | 7315 ± 368 | 332.5 |
| Ethanol (5%) + capric acid (0.5%) + 1-nonanol (0.5%) | 2100 ± 415 | 95.5 |
| Ethanol (10%) + capric acid (0.5%) + 1-nonanol (0.5%) | 1962 ± 77 | 89.2 |
| Ethanol (30%) + capric acid (0.5%) + 1-nonanol (0.5%) | 6312 ± 927 | 286.9 |
| Ethanol (20%) + capric acid (5%) + 1-nonanol (0.5%) | 3098 ± 398 | 140.9 |

### Test Example 3 (in vitro skin permeability test in human)

The procedure of Test Example 2 was repeated, except that a piece of the skin excised from human during operation ((1) skin containing only a straum corneum layer and an epidermal layer (thickness: approximately 100 µm) treated with NaBr, or (2) skin containing a horny layer, an epidermal layer, a dermal layer, and some fat layers (thickness: approximately 1 mm), which skin had been freeze-stored, and was thawed and fat in the skin was removed) was employed, and saline containing a solution of benzalkonium chloride (0.01%) was employed as a solution of a receiver side, to thereby calculate the cumulative skin permeation amount (Q) of compound A per unit area for 48 hours (µg/cm²), for comparison of skin permeability (n=2 or 3).

The results are shown in Tables 5 and 6. In Tables 5 and 6, the ratio of the cumulative skin permeation amount of compound A in the case of incorporation of a percutaneous absorption enhancer to that in the case without the enhancer is shown as "percutaneous absorption enhancing effect."

**Table 5**

| Percutaneous absorption enhancers | Q₄₈(µg/cm² ± Skin S.E.) | Percutaneous absorption enhancing effect |
|---|---|---|
| None | (2) 3 ± 1 | 1.0 |
| Capric acid (5%) | (2) 6048 ± 473 | 2016 |
| Capric acid (5%) + ethanol (10%) | (1) 8597 ± 4228 | 2666 |
| Capric acid (5%) + ethanol (20%) | (2) 10215 ± 2077 | 3405 |
| Capric acid (5%) + N-octyl-2-pyrrolidone (5%) + ethanol (20%) | (2) 20007 ± 4466 | 6669 |
| Capric acid (5%) + polyoxyethylene lauryl ether (5%) | (1) 6151 ± 3015 | 2050 |
| Capric acid (5%) + d-limonene (1%) | (2) 4249 ± 2061 | 1416 |
| Capric acid (5%) + d-limonene (5%) | (2) 5953 ± 742 | 1984 |
| Capric acid (5%) + d-limonene (1%) + ethanol (20%) | (2) 21434 ± 1236 | 7145 |
| Capric acid (5%) + d-limonene (1%) + ethanol (10%) | (2) 12713 ± 1067 | 4238 |
| Capric acid (5%) + benzyl alcohol (5%) | (1) 19427 ± 2475 | 6476 |
| Capric acid (5%) + benzyl alcohol (20%) | (1) 15256 | 5086 |
| Capric acid (5%) + benzyl alcohol (1%) | (2) 6922 ± 2068 | 2717 |
| Capric acid (5%) + benzyl alcohol (5%) + ethanol (20%) | (2) 14081 ± 2091 | 4694 |
| Capric acid (5%) + benzyl alcohol (5%) + d-limonene (1%) | (2) 19572 ± 2350 | 6657 |
| Capric acid (5%) + benzyl alcohol (5%) + d-limonene (1%) + ethanol (20%) | (2) 22555 ± 445 | 7518 |
| Capric acid (0.5%) + 1-nonanol (0.5%) + ethanol (20%) | (2) 10427 ± 3756 | 3476 |

**Table 6**

| Percutaneous absorption enhancers | Skin | Q₄₈(µg/cm² ± S.E.) | Percutaneous absorption enhancing effect |
|---|---|---|---|
| Capric acid (0.5%) + 1-nonanol (0.5%) ethanol(20%) | (1) | 11643 ± 6969 | 3881 |
| Capric acid (5%) + 1-nonanol (1%) + ethanol (20%) | (2) | 11408 ± 2888 | 3803 |
| Capric acid (5%) + benzyl alcohol (5%) + 1-nonanol (1%) | (2) | 13848 ± 1907 | 4616 |
| Capric acid (5%) + benzyl alcohol (5%) + 1-nonanol (1%)+ ethanol(20%) | (2) | 23032 ± 3111 | 7677 |
| Capric acid (5%) + benzyl alcohol (5%) + d-limonene (1%) + ethanol (20%) + 1-nonanol (1%) | (2) | 21929 ± 52 | 7310 |
| 1-Nonanol (1%) + benzyl alcohol (5%) | (2) | 3657 ± 2891 | 1286 |
| 1-Nonanol (1%) + benzyl alcohol (5%) + ethanol (20%) | (2) | 15156 ± 1451 | 5052 |
| 1-Nonanol (1%) + benzyl alcohol (5%) + ethanol (10%) | (2) | 7420 ± 1601 | 2473 |
| Benzyl alcohol (5%) + d-limonene (1%) | (2) | 12306 ± 2234 | 4102 |
| Benzyl alcohol (5%) + d-limonene (5%) | (2) | 10473 ± 3743 | 3491 |
| Benzyl alcohol (5%) + d-limonene (1%) + ethanol (20%) | (2) | 27242 ± 2162 | 9081 |

As is apparent from Tables 1 to 6, the skin permeability of an aromatic amidine derivative (I), a salt of the derivative, a solvate of the derivative, or a solvate of a salt of the derivative is drastically enhanced when employed in combination with a percutaneous absorption enhancer.

### Example 1

Compound A (1.2 g), 1,3-butylene glycol (1.2 g), benzalkonium chloride (0.04 g), purified water (6.4 g), an acrylic emulsion-type adhesive (product name: Nikkasol TS-620, product of Nippon Carbide Industries Co., Inc.) (15.8 g), and capric acid (0.36 g) serving as a percutaneous absorption enhancer were mixed and stirred. While water was appropriately added to the resultant mixture, the mixture was further stirred, to thereby adjust the viscosity thereof. The thus-obtained formulation was applied to a polyester film and dried, to thereby obtain a plaster containing a percutaneous absorption enhancer.

### Example 2

1,3-Butylene glycol (1.2 g), benzalkonium chloride (0.04 g), purified water (6.4 g), an acrylic emulsion-type adhesive (product name: Nikkasol TS-620, product of Nippon Carbide Industries Co., Inc.) (13.87 g) were mixed and stirred. To the resultant mixture, capric acid (1.0 g) serving as a percutaneous absorption enhancer, polyoxyethylene lauryl ether (1.0 g), and a solution in which compound A (1.2 g) was dissolved or dispersed in purified water (6.4 g) were added, and the resultant mixture was stirred. Furthermore, water was appropriately added to the mixture, to thereby adjust the viscosity thereof. The thus-obtained formulation was applied to a polyester film and dried, to thereby obtain a plaster containing a percutaneous absorption enhancer.

### Example 3

N-[4-[(1-Acetimidoyl-4-piperidyl)oxy]phenyl]-N-[(7-amidino-2-naphthyl)methyl]sulfamoylacetic acid dihydrochloride (hereinafter referred to as "compound B") (1.2 g), 1,3-butylene glycol (1.2 g), benzalkonium chloride (0.04 g), and purified water (6.4 g) were mixed and stirred, to thereby obtain a preparatory solution. Subsequently, a mixture of an acrylic emulsion-type adhesive (product name: Nikkasol TS-620, product of Nippon Carbide Industries Co., Inc.) (15.8 g) and lauryldimethylamine oxide (0.36 g) serving as a percutaneous absorption enhancer was added to the preparatory solution. While water was appropriately added to the resultant mixture, the mixture was further stirred, to thereby adjust the viscosity thereof. The thus-obtained formulation was applied to a polyester film and dried, to thereby obtain a plaster containing a percutaneous absorption enhancer.

### Example 4

A carboxyvinyl polymer (1.5 wt.%) and triethanolamine (2.0 wt.%) were dissolved in purified water (66.5 wt.%), and the resultant solution was allowed to stand for 12 hours or more, to thereby sufficiently swell a substrate. Separately, compound A (5 wt.%) and capric acid (5 wt.%) serving as a percutaneous absorption enhancer were dissolve or dispersed in ethanol (20 wt.%), and the resultant solution was gradually added to the swollen substrate. The resultant mixture was stirred by use of a magnetic stirrer for approximately eight hours, to thereby prepare an external-use gel formulation containing a percutaneous absorption enhancer.

### Example 5

A carboxyvinyl polymer (1.5 wt.%) and triethanolamine (2.0 wt.%) were dissolved in purified water (81.5 wt.%), and the resultant solution was allowed to stand for 12 hours or more, to thereby sufficiently swell a substrate. Separately, compound A (5 wt.%) and capric acid (5 wt.%) serving as a percutaneous absorption enhancer were dissolve or dispersed in benzyl alcohol (5.0 wt.%), and the resultant solution was gradually added to the swollen substrate. The resultant mixture was stirred by use of a magnetic stirrer for approximately eight hours, to thereby prepare an external-use gel formulation containing a percutaneous absorption enhancer.

### Example 6

To a mixer in which a temperature for kneading was kept at 40°C, purified water (49 wt.%), sodium edetate (0.1 wt.%), light silicic acid anhydride (2 wt.%), and titanium oxide (0.3 wt.%) were sequentially added, to thereby dissolve and disperse these substances in purified water. To the resultant solution, a carboxyvinyl polymer (1 wt.%) and carboxymethyl cellulose sodium (1.0 wt.%) were gradually added, and the mixture was stirred for 10 minutes, to thereby obtain a solution. Separately, compound A (5 wt.%), capric acid (5.0%) serving as a percutaneous absorption enhancer, benzyl alcohol (5.0%), and d-limonene (1.0 wt.%) were dissolved or dispersed in glycerin (11.5 wt.%). The resultant solution was added to the above-obtained solution, and the mixture was stirred for 10 minutes, to thereby obtain a paste solution. The paste solution was transferred into a kneader which was heated to 40°C. Subsequently, tartaric acid (1 wt.%) in purified water (2 wt.%) was added to paste solution and the mixture was kneaded for five minutes. To the resultant mixture, a solution in which a dried aluminum hydroxide gel (0.1 wt.%) and partially-neutralized polyacrylic acid (6 wt.%) were dispersed in glycerin (16 wt.%) was gradually added, and the mixture was kneaded for 40 minutes, to thereby obtain a composition. The composition was extended onto a support, to thereby obtain a cataplasm.

### Example 7

Propylene glycol (2 wt.%), polyoxyethylene alkyl ether (product name: Nikkol BC20TX, product of Nikko Chemicals Co., Ltd.) (0.6 wt.%), sorbitan monooleate (product name: Nikkol SO-10, product of Nikko Chemicals Co., Ltd.) (1.4 wt.%), ethanol (4.5 wt.%) serving as a percutaneous absorption enhancer, sodium lauryl sulfate (0.5 wt.%), and water (72 wt.%) were mixed, to thereby prepare a substrate of an external-use solution agent. To the substrate, dimethylstearylamine (1.2 wt.%), d-limonene (2.8 wt.%) serving as a percutaneous absorption enhancer, benzyl alcohol (10 wt.%), and compound A (5 wt.%) were added while the mixture was aggressively stirred by use of a homogenizer, to thereby obtain an external-use solution agent.

### Example 8

Polyoxyethylene hydrogenated castor oil 60 (5 wt.%) and hydrogenated rapeseed oil (27.5 wt.%) were heated and dissolved each other. After the resultant mixture was cooled, isopropyl myristate (47 wt.%) was gradually added to the mixture and sufficiently mixed together. To the resultant mixture, a solution of compound B (5 parts by weight) in isopropanol (4.5 wt.%), capric acid (5.0 wt.%) serving as a percutaneous absorption enhancer, benzyl alcohol (5.0 wt.%), and 1-nonanol (1.0 wt.%) was added, and the mixture was sufficiently kneaded until it became homogeneous, to thereby obtain an ointment.

### Example 9

Methylparaben (0.3 wt.%) was dissolved in purified water (29.1 wt.%) under heat, and compound A (5 wt.%), glycerin monocaprylate (0.5 wt.%), and propylene glycol (10 wt.%) were added to the resultant solution. Subsequently, dimethylacetamide (2 wt.%), benzyl alcohol (5.0 wt.%) serving as a percutaneous absorption enhancer, and d-limonene (1.0 wt.%) were sequentially added to the resultant solution. Separately, white paraffin (25 wt.%) and stearyl alcohol (22.1 wt.%) were dissolved each other under heat. The solution was mixed with the above-obtained solution, and the mixture was emulsified and cooled, to thereby obtain a cream.

### Example 10

A carboxyvinyl polymer (1 wt.%) was swollen in purified water (23 wt.%). To the solution, a mixture of propylene glycol (30 wt.%), triethyl citrate (20 wt.%), compound A (5 wt.%), and 2-hydroxy-4-methoxybenzophenone (0.5 wt.%) was added, and mixed and stirred. To the resultant mixture, a solution of benzyl alcohol (5.0 wt.%) serving as a percutaneous absorption enhancer, d-limonene (1.0 wt.%), and diisopropanolamine (1.1 wt.%) in purified water (13.4 wt.%) was added, and the resultant mixture was sufficiently stirred until it became uniform, to thereby obtain a gel.

Subsequently, the thus-obtained gel (4.0 g) was sealed in a drug-reservoir layer having an ethylene-vinyl acetate copolymer resin serving as a drug-releasing layer, the drug-reservoir layer being backed by a polyester film support having an acrylic adhesive layer provided at a peripheral portion of a pressure-sensitive adhesive surface, and polyethylene terephthalate film serving as a peelable liner being further provided on the pressure-sensitive adhesive surface, to thereby obtain a reservoir-type patch.

### Industrial Applicability

According to the present invention, there can be obtained a percutaneously absorbable medicinal composition which has a high percutaneous absorbability, can maintain an available blood level for a prolonged period, and has antithrombotic and anticoagulant effects.

## Claims

1. A medicinal composition for percutaneous administration comprising a percutaneous absorption enhancer and an aromatic amidine derivative represented by the following formula (1), a salt of the derivative, a solvate of the derivative, and a solvate of the salt of the derivative: 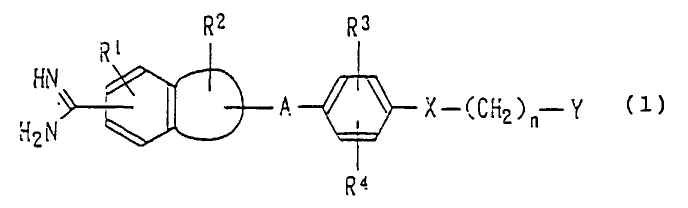 [wherein
R¹ represents a hydrogen atom or a lower alkoxyl group;
R² represents a hydrogen atom, a lower alkyl group, a lower alkoxyl group, a carboxyl group, an alkoxycarbonyl group, a carboxyalkyl group, or an alkoxycarbonylalkyl group;
R³ represents a hydrogen atom, a carboxyl group, an alkoxycarbonyl group, a carboxyalkyl group, an alkoxycarbonylalkyl group, a carboxyalkoxyl group or an alkoxycarbonylalkoxyl group;
R⁴ represents a hydrogen atom, a halogen atom, an amino group, a cyano group, a nitro group, a hydroxyl group, a lower alkyl group, or a lower alkoxyl group;
n represents an integer 0 to 4; and
A represents a C1-C4 alkylene group optionally substituted by one or two hydroxyalkyl groups, carboxyl groups, alkoxycarbonyl groups, carboxyalkyl groups, or alkoxycarbonylalkyl groups, or a group represented by the following formula; 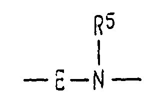 {wherein
E represents a lower alkylene group or a carbonyl group and R⁵ represents a hydrogen atom or a group represented by formula -D-W-R⁶ (wherein D is a group represented by 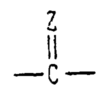 (wherein
Z is an oxygen atom or a sulfur atom), or a group represented by 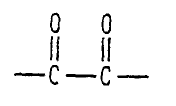 or a sulfonyl group;
W represents a single bond or a group represented by -NR⁷-(wherein R⁷ represents a hydrogen atom, a carbamoyl group, a lower alkoxycarbonyl group, a mono- or di-lower alkylaminocarbonyl group, a lower alkylsulfonyl group, a mono- or di-lower alkylaminothiocarbonyl group, a lower alkyl group which may have a substituent, or a lower alkanoyl group which may have a substituent);
R⁶ represents a hydroxyl group, a lower alkoxyl group, a lower alkyl group which may have a substituent, an aryl group which may have a substituent, or a heteroaryl group which may have a substituent)};
X represents a single bond, an oxygen atom, a sulfur atom, or a carbonyl group;
Y represents a saturated or unsaturated 5- or 6-membered heterocyclic or cyclic hydrocarbon group which may have a substituent, an amino group which may have a substituent, or an aminoalkyl group which may have a substituent; and the group represented by 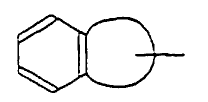 represents a group selected from among indolyl, benzofuranyl, benzothienyl, benzimidazolyl, benzoxazolyl, benzothiazolyl, naphthyl, tetrahydronaphthyl, or indanyl].

2. A composition according to claim 1, wherein the percutaneous absorption enhancer is one or more species selected from among an alcohol, a polyhydric alcohol, a higher alkane, a higher fatty acid, a higher fatty acid ester, a terpene, an alkyl sulfate ester, an alkylamine oxide, a carboxybetaine, a polyoxyalkylene alkyl ether, a sulfoxide, and an amide.

3. A composition according to claim 2, wherein the alcohol is a C2-C16 saturated aliphatic alcohol or a C7-C14 arylalkanol.

4. A composition according to claim 2, wherein the polyhydric alcohol is a C2-C4 alkylene glycol, glycerin, polyethylene glycol, polypropylene glycol, polyglycerin, or sorbitan.

5. A composition according to claim 2, wherein the higher alkane is a C6-C12 alkane.

6. A composition according to claim 2, wherein the higher fatty acid is a C6-C18 saturated or unsaturated fatty acid.

7. A composition according to claim 2, wherein the higher fatty acid ester is an ester of a polyhydric alcohol and a C6-C24 fatty acid.

8. A composition according to claim 2, wherein the terpene is selected from among cineole, 1-menthol, menthone, d-limonene, and nerolidol.

9. A composition according to claim 2, wherein the alkyl sulfate ester is a C6-C24 alkyl sulfate ester salt.

10. A composition according to claim 2, wherein the alkylamine oxide is a C6-C24 alkyldimethylamine oxide.

11. A composition according to claim 2, wherein the carboxybetaine is C6-C24 alkyldimethylaminoacetic acid.

12. A composition according to claim 2, wherein the polyoxyalkylene alkyl ether is polyoxyethylene alkyl ether.

13. A composition according to claim 2, wherein the sulfoxide is dialkyl sulfoxide, and the amide is N,N-dialkylformamide, N,N-dialkylacetamide, N,N-dialkyltoluamide, N-hydroxyalkylactamide, hydroxy or N-alkylpyrrolidone.

14. A composition according to claim 2, wherein the percutaneous absorption enhancer is one or more species, used singly or in combination, selected from among C2-C4 alcohols, C7-C12 arylalkanols, C6-C18 saturated aliphatic alcohols, polyhydric alcohols, C6-C16 fatty acids, terpenes, polyhydric alcohol mono C6-C16 fatty acid esters, C6-C24 alkyl sulfate salts, C6-C24 alkyldimethylamine oxides, C6-C24 alkyldimethylaminoacetic acids, polyethylene glycol C6-C24 alkyl ethers, pyrrolidone, N-alkylpyrrolidones, N,N-dialkylformamide, and N,N-dialkyltoluamides.

15. A composition according to claim 2, wherein the percutaneous absorption enhancer is one or more species, used singly or in combination, selected from among ethanol, isopropanol, benzyl alcohol, ethylene glycol, propylene glycol, glycerin, 1-nonanol, 1-decanol, stearyl alcohol, caproic acid, caprylic acid, capric acid, d-limonene, cineole, propylene glycol monocaprylate, glycerin monocaprylate, glycerin monocaprate, lauryldimethylamine oxide, dodecyl-N,N-dimethylaminoacetic acid, sodium lauryl sulfate, polyoxyethylene lauryl ether, N,N-diethyltoluamide, 2-pyrrolidone, and N-octyl-2-pyrrolidone.

16. A composition according to claim 2, wherein the percutaneous absorption enhancer is one or more species, used singly or in combination, selected from among C2-C4 alcohols, C7-C12 arylalkanols, C6-C18 saturated aliphatic alcohols, C6-C16 fatty acids, terpenes, pyrrolidone, and N-alkylpyrrolidone.

17. A composition according to claim 2, wherein the percutaneous absorption enhancer is one or more species, used singly or in combination, selected from among ethanol, capric acid, benzyl alcohol, 1-nonanol, N-octyl-2-pyrroridone, and d-limonene.

18. A composition according to any one of claims 1 through 17, wherein, in formula (1), the group represented by 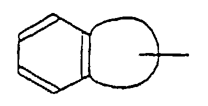 is a group selected from among benzofuranyl, benzimidazolyl, indolyl, benzothienyl, benzothiazolyl, naphthyl, and tetrahydronaphthyl.

19. A composition according to any one of claims 1 through 18, wherein, in formula (1), the saturated or unsaturated 5- or 6-membered heterocyclic group is a group containing 1 or 2 nitrogen or oxygen atoms as hetero atoms.

20. A composition according to any one of claims 1 through 18, wherein, in formula (1), the saturated or unsaturated 5- or 6-membered heterocyclic group is a pyrrolidinyl group or a piperidyl group.

21. A composition according to any one of claims 1 through 17, wherein the aromatic amidine derivative, the salt of the derivative, the solvate of the derivative, or the solvate of the salt of the derivative is a compound, a salt thereof, or a solvate of the compound or the salt selected from the group consisting of the following members:
2-[4-[((3S)-1-acetimidoyl-3-pyrrolidinyl)oxy]phenyl]-3-(7-amidino-2-naphthyl)propionic acid,
(+)-2-[4-[((3S)-1-acetimidoyl-3-pyrrolidinyl)oxy]phenyl]-3-(7-amidino-2-naphthyl)propionic acid,
(2S)-2-[4-[((3S)-1-acetimidoyl-3-pyrrolidinyl)oxy]phenyl]-3-(7-amidino-2-naphthyl)propionic acid,
(2R)-2-[4-[((3R)-1-acetimidoyl-3-pyrrolidinyl)oxy]phenyl]-3-(7-amidino-2-naphthyl)propionic acid,
2-[4-[(1-acetimidoyl-4-piperidyl)oxy]phenyl]-3-(7-amidino-2-naphthyl)propionic acid,
(+)-2-[4-[(1-acetimidoyl-4-piperidyl)oxy]phenyl]-3-(7-amidino-2-naphthyl)propionic acid,
2-[4-[(1-acetimidoyl-4-piperidyl)oxy]phenyl]-3-(5-amidinobenzo[b]thien-2-yl)propionic acid,
2-[4-[((2S)-1-acetimidoyl-2-pyrrolidinyl)methoxy]phenyl]-3-(5-amidinobenzo[b]thien-2-yl)propionic acid,
(+)-2-[4-[((2S)-1-acetimidoyl-2-pyrrolidinyl)methoxy]phenyl]-3-(5-amidinobenzo[b]thien-2-yl)propionic acid,
3-[4-[((3S)-1-acetimidoyl-3-pyrrolidinyl)oxy]phenyl]-4-(5-amidinobenzo[b]thien-2-yl)butyric acid,
2-[4-[((3S)-1-acetimidoyl-3-pyrrolidinyl)oxy]phenyl]-3-(6-amidino-1-ethyl-2-indolyl)propionic acid,
2-[4-[((3R)-1-acetimidoyl-3-pyrrolidinyl)oxy]phenyl]-3-(6-amidino-1-ethyl-2-indolyl)propionic acid,
2-[4-[(1-acetimidoyl-4-piperidyl)oxy]phenyl]-3-(6-amidino-1-ethyl-2-indolyl)propionic acid,
N-[4-[(1-acetimidoyl-4-piperidyl)oxy]phenyl]-N-[(7-amidino-2-naphthyl)methyl]-N'-methylsulfamide,
ethyl N-[N-4-[(1-acetimidoyl-4-piperidyl)oxy]phenyl]-N-[(7-amidino-2-naphthyl)methyl]sulfamoyl]carbamate,
4-[N-4-[(1-acetimidoyl-4-piperidyl)oxy]phenyl]-N-[(7-amidino-2-naphthyl)methyl]sulfamoyl]benzoic acid,
N-[4-[(1-acetimidoyl-4-piperidyl)oxy]phenyl]-N-[(7-amidino-2-naphthyl)methyl]sulfamoylacetic acid,
ethyl N-[N-[4-[(1-acetimidoyl-4-piperidyl)oxy]phenyl]-N-[(7-amidino-2-naphthyl)methyl]sulfamoyl]glycinate,
N-[N-4-[(1-acetimidoyl-4-piperidyl)oxy]phenyl]-N-[(7-amidino-2-naphthyl)methyl]sulfamoyl]-N-ethoxycarbonylglycine, and
N-[N-4-[(1-acetimidoyl-4-piperidyl)oxy]phenyl]-N-[(7-amidino-2-naphthyl)methyl]sulfamoyl]glycine.

22. A composition according to any one of claims 1 through 17, wherein the aromatic amidine derivative, the salt of the derivative, the solvate of the derivative, or the solvate of the salt of the derivative is selected from the group consisting of the following members:
(2S)-2-[4-[((3S)-1-acetimidoyl-3-pyrrolidinyl)oxy]phenyl]-3-(7-amidino-2-naphthyl)propionic acid hydrochloride pentahydrate,
(+)-2-[4-[(1-acetimidoyl-4-piperidyl)oxy]phenyl]-3-(7-amidino-2-naphthyl)propionic acid dihydrochloride,
(+)-2-[4-[((2S)-1-acetimidoyl-2-pyrrolidinyl)methoxy]phenyl]-3-(5-amidinobenzo[b]thien-2-yl)propionic acid dihydrochloride,
ethyl N-[N-[4-[(1-acetimidoyl-4-piperidyl)oxy]phenyl]-N-[(7-amidino-2-naphthyl)methyl]sulfamoyl]glycinate dihydrochloride,
N-[N-4-[(1-acetimidoyl-4-piperidyl)oxy]phenyl]-N-[(7-amidino-2-naphthyl)methyl]sulfamoyl]glycine dihydrochloride, and
N-[4-[(1-acetimidoyl-4-piperidyl)oxy]phenyl]-N-[(7-amidino-2-naphthyl)methyl]sulfamoylacetic acid dihydrochloride.
